# EUROPEAN PATENT SPECIFICATION

(11) **EP 0 482 571 B1**
(45) Date of publication and mention of the grant of the patent: **26.02.1997**
(21) Application number: 91117977.8
(22) Date of filing: 22.10.1991
(51) Int. Cl.: G01N 33/546, G01N 33/53, G01N 15/14, G01N 33/551

(54) **Assay of substances using flow cytometry**
Bestimmung von Substanzen mittels durchfluss Zytometrie
Essai de substances utilisant cytometrie en flux

(30) Priority: 22.10.1990 JP 285588/90; 22.11.1990 JP 319171/90
(43) Date of publication of application: 29.04.1992
(73) Proprietor: SUMITOMO ELECTRIC INDUSTRIES, LTD., Osaka-shi, Osaka 541 (JP)
(72) Inventor: Kishimoto, Toshihiko, c/o Osaka Works of Sumitomo, Konohana-ku, Osaka (JP); Niwa, Shin-ichiro, c/o Osaka Works of Sumitomo, Konohana-ku, Osaka (JP); Nakabayashi, Makoto, c/o Osaka Works of Sumitomo, Konohana-ku, Osaka (JP); Shirai, Toshikazu, Kokubunji-shi, Tokyo (JP); Hirose, Sachiko, Shibuya-ku, Tokyo (JP); Tsurui, Hiromichi, Nerima-ku, Tokyo (JP)
(74) Representative: Hansen, Bernd, Dr. Dipl.-Chem.

(56) References cited:
- EP-A- 0 135 051
- FR-A- 2 627 286
- FR-A- 2 638 849
- GB-A- 2 029 571
- US-A- 4 665 020

## Description

The present invention relates to an assay useful in various fields of technology including immunology, molecular biology, diagnosis and the like. More particularly, it relates to a method for qualitatively or quantitatively measuring a substance to be detected in a sample by means of flow cytometry, wherein said substance is bound to a microparticle through a second substance which is immobilized to-said particle and capable of binding to the substance specifically.

Reliable and efficient assay for a given substance in a sample has been continuously needed in various fields of technology including medicine, chemistry, agriculture and the like, and there have been provided various methods before the present invention. A group of widely known methods employs a specific reaction between a substance to be detected (hereinafter, referred to as an analyte) and a second substance, for example, a reaction between an antigen and its antibody. These methods are generally carried out by immobilizing said second substance to an insoluble carrier, contacting a sample containing the analyte to the immobilized second substance, separating the carrier from the reaction mixture and determining the presence or absence, or concentration of said analyte captured on the carrier through the second substance. Typical assays of this type are immunoassays such as Enzyme-Linked Immunosorbent Assay (ELISA). Outline of such a conventional immunoassay is described below.

A plastic plate having multiple wells is often used as the solid carrier. To each of the wells is added a solution containing an antigen and the plate is kept overnight at about 4°C or for about 3 hours at room temperature. After the solution is discarded, the wells are blocked in order to prevent any unspecific adsorptions. The blocking is carried out by filling the wells with a solution of a blocking agent such as skim milk, standing for several hours at room temperature or overnight at about 4°C, and washing thoroughly with PBS buffer. To the blocked wells is added a sample solution suspected to contain the antibody to be assayed, and the plate is kept for several hours and washed thoroughly, which is followed by the addition of an antibody labeled with an appropriate signal-producing substance such as enzyme, fluorogenic substance, or the like. The plate is then allowed to stand for several hours. After washing the plate, the signal produced in each well is observed by any of known methods. When the label is a fluorogenic substance, the observation is carried out by adding a suitable buffer to the wells and determining the level of fluorescence with fluorophotometer. When the label is an enzyme, a solution of a substrate is added to each well and allowed to react for a while. Thereafter, the reaction is stopped and the absorption of light caused by the products of enzymatic reaction is measured with spectrophotometer.

FR-A-2638849 teaches the labelling of particles by a fluorescent reagent. When particles are labeled, one cannot conduct exact quantitative determination because it is very difficult to attach the same amount of a fluorescent to every particle. Thus, the presence of a substance to be determined can only be detected in a qualitative manner.

The method disclosed in US-A-4,665,020 present an "indirect method". That is, an antigen in a sample solution is first contacted with a carrier on the surface of which an antibody thereto has been immobilized. Smaller fluorescent particles coated with the antigen are contacted with said carrier whereby free antibodies on the carrier bind to the smaller fluorescent particles coated with the antigen. The amount of such fluorescent particles bound to the carrier through the antibody is determined by flow cytometry.

GB-A-2029571 relates to sandwich-type specific binding (for example, immunological) assays.

EP-A-0 135 051 describes immunological assays for the determination of anti-DNA antibodies using immobilized DNA.

FR-A-2627286 discloses a method for the simultaneous performance of several assays (that is, antigen/antibody reactions) without specifying the particles used.

As stated above, the existing methods generally use, as a solid carrier, a plastic product in the form of microtiter plate, tube or the like. However, such a plastic solid carrier does not always afford reliable data because of its tendency to non-specific binding. Thus, unspecific binding or attachment of various substances to the carrier makes it difficult to establish a uniformity of binding between the carrier and a substance to be immobilized and causes undesirable background effects. Furthermore, when a microtiter plate is used as a solid carrier, each well gives just one datum which differs from well to well, resulting in the inefficiency of the experiment and unreliability of the data.

One solution of the above problems lies in bringing the binding between the carrier and the substance to be immobilized more specific, thereby eliminating the background effects and reducing the variation of data.

The present inventors have now found that the above problems can be solved by contacting a sample solution containing an analyte with particulate carriers to which a second substance capable of specifically binding to said analyte has been immobilized, and qualitatively or quantitatively detecting the analyte bound to the particles through the second substance by means of flow cytometry.

Thus, the present invention provides a method for qualitatively or quantitatively measuring an antibody A in a sample solution, which in principle comprises the steps as outlined in claim 1.

For the purpose of the invention, as disclosed and claimed herein, the following terms are defined below.

The term "substance A" or "analyte" refers to an antibody.

The term "substance B " or "second substance" refers to an antigen which can specifically react and bind with the substance A or analyte and can bind to a microparticle of silica gel used as a carrier. A combination of an antigen and its specific monoclonal antibody is preferable.

The term "sample solution" means a solution which is known or suspected to contain the substance A. Examples of sample solutions include blood, urine, body fluid, cultured broth and the like.

The apparatus used for the flow cytometry is commercially available and any of existing devices can be used in the present method. The determination by flow cytometry is effected basically by passing a suspension of particulate substances to be detected, for example cells or chromosomes, through a fluid system at high speed while detecting the photoelectric signals from individual particle on detector. The detection is carried out by irradiating a laser beam to suspended particles moving at high speed and converting the optical properties of each particle into photoelectric signals. The optical property of the particle is generally provided by appropriate signal-producing systems. Such signal-producing systems involve the production of detectable signals such as absorption, scattering or emission of light. Examples of signal-producing substances are fluorogenic or chromogenic compounds such as rhodamin, FITC (fluorescein isothiocyanate), acridine orange, propiodium iodite and the like. Such a signal-producing system can be attached to the microparticles by adding substances containing appropriate signal-producing substance associated to a molecule capable of binding to the substance A. In the present invention, said labeled substance is an appropriately labeled second antibody.

Examples of apparatus for the flow cytometry include FACS series distributed by Becton Dickinson, EPICS series distributed by Nippon Bunko, Ltd., Showa Denko, Ltd., Cor-tar Electronics, Ltd. and the like.

Microparticles to be used as a solid carrier may be any shape but preferably spherical of appropriate diameter for the determination by flow cytometry. The size of particles may vary depending on the size of a nozzle attached to the apparatus for flow cytometry. However, microparticles may be in the range of about 1 nm to 1000 µm, preferably about 1 nm to about 400 µm, more preferably, 100 nm to 100 µm, in diameter. Specifically, the average diameter of particles may be from about 1 nm to about 400 µm when the diameter of nozzle is between about 10 to about 1,000 µm, and the average diameter of particles may be from about 0.1 to about 100 µm when that of nozzle is between about 10 to about 500 µm. Especially preferred particles are those of about 0.1 to 100 µm in diameter.

The microparticles or particulate carriers comprise silica gel. Although these microparticles may be used without further treatment, they can be modified variously to make the immobilization of substances more stable and uniform. Particles may be subjected to a surface-finishing process by physical and/or chemical treatment, for example, ion-plating method and the like.

The immobilization of substance B to particles can be carried out according to known methods in the art. Although the procedure may vary depending on the substance B and the material of the particle, the substance B can be generally bound to particles covalently at a functional group present in the molecule composing said particle, for example, hydroxyl group, or the like. Thus, the immobilization can be accomplished by, for example, reacting an amino group of substance B and a hydroxyl group of carrier after the activation of the hydroxyl group using an activator such as trifluoroethanesulfonyl chloride or the like [R. Axen et al., Nature, vol. 214: pp.1302 (1967); T.H.Finlay et al., Anal. Biochem., vol. 87; pp.77 (1978); I.Matsumoto et al., J. Biochem., vol. 85: pp.1091 (1979); L.Sundberg, et al., J. Chromatogr., vol.90: pp.87 (1974); K.Nilson et al., Biochem. Biophys. Res. Commun., vol.102: pp.449 (1981); and J.Porath, Meth. Enzymol., vol.34: pp.27 (1974)]; reacting an amino or a carboxyl group of substance B and a carboxyl or an amino group of the carrier in the presence of a condensing reagent such as water-soluble carbodiimide (WSC; supplied by Wako Junyaku, Co. Ltd., Japan, and the like) [A. Tengblad, Biochem. J., vol.199: pp.297 (1981); M. Funabashi et al., Anal. Biochem., vol. 126: pp.414 (1982); G. Saccomani et al., J. Biochem., vol. 256: pp.12405 (1981); B. Belleau et al., J. Am. Chem. Soc., vol.90: pp.1651 (1968); and P. Cuartecasas et al., Biochemistry, vol.11: pp.2291 (1972)]; reacting amino group with an aldehyde group to yield a shiff base or a hydrazide and, if necessary, followed by reduction [Y. Ito et al., J. Biochem., vol.97: pp.1689 (1985); and R. J. Baues et al., J. Biol. Chem. vol.252: pp.57 (1977)]; or forming -S-S- bonds using thiol group [K.Brocklehurst et al., Biochem.J., vol.133: pp.573 (1973)]. In the present invention, the substance B is an antigen, especially a DNA molecule, and substance A is an anti-DNA monoclonal antibody, where said substance B is bound to a microparticle of silica gel by terminal-immobilization.

The immobilized substance B is useful in the determination of presence or absence, or concentration of substance A in a sample. Accordingly, in another embodiment of the invention, it is provided an- immobilized substance B useful for the qualitative or quantitative determination of substance A by flow cytometry, wherein said substance B can react and bind with said substance A specifically, and the solid carrier is microparticles.

As mentioned above, the present method allows to detect substance A by associating said substance A to a microparticle with the aid of substance B which is immobilized to the particle and is capable of reacting specifically with the substance A, and subjecting the microparticle to flow cytometry. In the preferred embodiment of the present invention, the substance B is immobilized uniformly to a particulate carrier through a covalent bond between functional groups such as amino, hydroxyl, carboxyl, thiol groups and the like. As a particulate carrier one of those used for HPLC i.e. silica gel is employed because it hardly generates any non-specific binding, which results in the reduction of background effects and the improvement of accuracy of measurement.

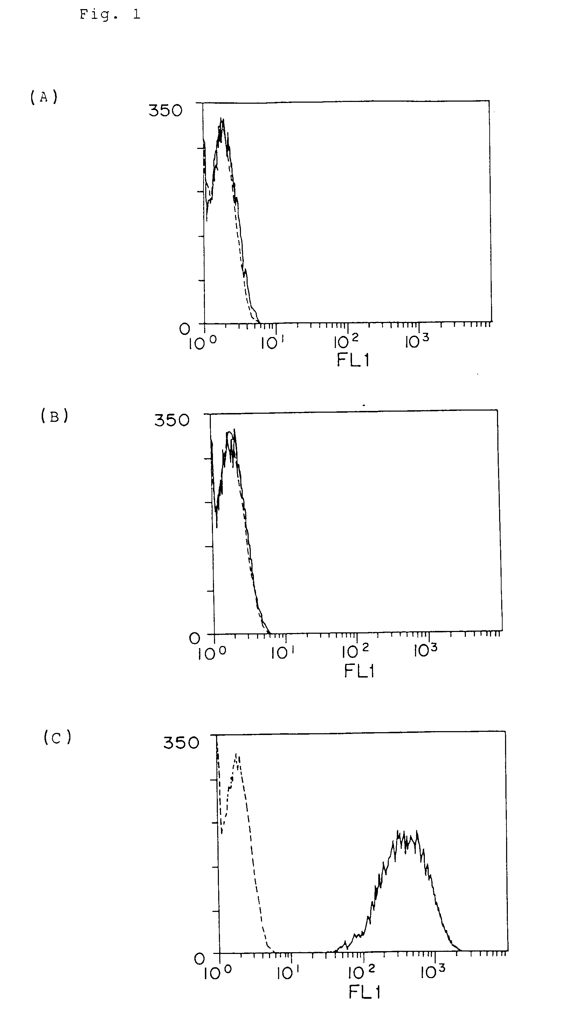

In comparison with the currently used ELISA, where a microtiter plate is used as a solid carrier, the present assay has following advantages. The present assay is more efficient than ELISA because one particle in the present assay can be regarded to be equivalent to one well in ELISA and almost 10⁵ of particles can be measured by one trial. Furthermore, abnormal data are omitted from the results and the background effect is reduced as previously mentioned. Thus, the present method affords about 10 ⁴⁻⁵ times of data with high reliability and less errors within almost the same detection time compared with ELISA. According to the present method, the strength of affinity and reactivity are obtainable as detection patterns as shown in the accompanying drawings, Figures 1 to 8.

In the Figures:

Figure 1 shows the detection patterns of anti-DNA antibodies using a mixture of particulate carriers to which DNA fragments (a) and (b), as described in Example 1, are immobilized as single-stranded DNA, separately. The number of particles are plotted on the ordinate and the level of fluorescence on the abscissa. The solid lines represent the detection pattern obtained in the experiments conducted using antibody-containing samples and the dotted lines represent those obtained in the control experiments conducted using antibody-free samples. The panel A depicts the binding effect of IgG, the panel B depicts that of IgM, and the panel C depicts that of anti-DNA monoclonal antibody, to the DNA-immobilized particulate carriers. The order of the total number of particles shown on the ordinate is 10,000.

Figure 1 indicates that neither of the non-specific antibodies IgG and IgM which lack affinity to the DNA to be detected, causes the shift of peak (panels A and B, respectively), whereas the specific monoclonal antibody causes a significant shift of peak (panel C), demonstrating that the present method allows the detection of specific antibody correctly.

Figures 2 is similar to Figure 1 except that the carriers to which DNA (a) and (b) are immobilized in the form of double-stranded DNA are used.

Figure 3 is similar to Figure 1 except that the carriers to which DNA (c) and (d), as described in Example 1, are immobilized as single-stranded DNA are used.

Figure 4 is similar to Figure 3 except that the carriers to which DNA (c) and (d) are immobilized as double-stranded DNA are used.

Figure 5 is similar to Figure 1 except that the carriers to which DNA (e) and (f), as described in Example 1, are immobilized as single-stranded DNA are used.

Figure 6 is similar to Figure 5 except that the carriers to which DNA (e) and (f) are immobilized as double-stranded DNA are used.

Figure 7 is similar to Figure 1 except that lysozyme is immobilized to particulate carriers and anti-lysozyme monoclonal antibody is used as an specific antibody.

Figure 8 is similar to Figure 1 except that panels A and C show the results obtained using carriers to which DNA (a) and (b), as shown in Example 3, are immobilized as single-stranded DNA, while the panels (B) and (D) show the results obtained using carriers to which double-stranded DNA consisting of (a) and (b) are immobilized. Additionally, the panels A and B depict the binding effect of IgG and the panels C and D depict the binding effect of anti-DNA monoclonal antibody.

In the present assay, the variation of data, that is, the variation of level of fluorescence from particle to particle, is reflected as the breadth of peak. Further, each particle can be regarded to be equivalent to one well of the currently used ELISA. This means that, according to the present method, about 10⁴ to 10⁵ times of data can be obtained by only one experiment in comparison with ELISA. The data which are shown as a peak are highly reliable and allow one to evaluate the affinity between two substances, for example, an antigen and antibody, correctly and efficiently.

Although the present assay is useful in various fields, it is especially useful in clinical experiments, immunology, biochemistry and the like for the purpose of detection or diagnosis of various diseases and/or detection of antibody, specific substances and the like.

Following Examples further illustrate and detail the invention disclosed, but should not be construed to limit the invention.

### Example 1

### DNA-IMMOBILIZED CARRIER

### 1. Preparation of DNA-Immobilized Particulate Carrier

### A. Preparation of activated microparticles for the immobilization of DNA

As a solid carrier for the immobilization of DNA, silica gel is activated with trifluoroethanesulfonyl chloride (tresyl chloride: K & K, Co. Ltd.). SIMPACK diol 300 (average particle size = 5µm in diameter; SHIMAZU, Co. Ltd.) (1 g) is put into a No. 5 mesh glass filter and washed with acetone (20 ml), and then dry acetone (20 ml) by filtering with suction. The gel is taken in a 25 ml round bottle flask, and dry acetone (1 ml), pyridine (100 µl) and a small stirrer chip are added thereto. To the flask is added dropwise 100 to 200 µl of tresyl chloride gradually over about 1 minute while stirring vigorously and keeping away from air by introducing dry nitrogen gas. After the round bottle flask is closed with a plug, reaction is continued further 20 minutes under cooling and stirring gently. When the reaction completes, the mixture is filtered by No. 5 mesh glass filter and washed with 25 ml each of dry acetone, acetone/5mM HCl (1:1), 5 mM HCl, and acetone, successively, and acetone is evaporated. Thus obtained tresyl-activated SIMPAK diol 300 are collected in a Kjeldahl flask. After evaporation of the acetone under reduced pressure, the resultant activated silica gel is sealed in the flask until use.

### B. Preparation of DNA

The following DNA fragments were synthesized using DNA Synthesizer A-391EP PCR MATE (Applied Biosystems Inc. (ABI)). In the following fragments, (a) is a 27-mer to be immobilized and (b) is a complementary strand thereof, (c) is a 24-mer to be immobilized and (d) is a complementary strand thereof, and (e) is a 21-mer to be immobilized and (f) is a complementary strand thereof. 5′ X ACT TTC TGT TGC TTC ATC TTT GCA GAG 3′ (a) 5′ CTC TGC AAA GAT GAA GCA ACA GAA AGT (b) 5′ X GTC TGG GAA AAA CCC CCT TTG AGT (c) 5′ ACT CAA AGG GGG TTT TTC CCA GAC (d) 5′ X AGG TGA ATT TCT TAA ACA GCT (e) 5' AGC TGT TTA AGA AAT TCA CCT (f) X: Aminolink-2 (ABI, Co. Ltd.), which is a reagent used in the DNA synthesis to introduce an amino group at the 5'-terminus of a DNA molecule and is shown by the formula:

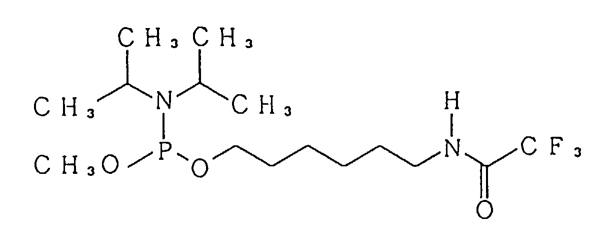

The synthesis and purification of the above DNA fragments were carried out according to the teaching of the manufacturer.

### C. Immobilization of DNA to Carrier

Immobilization of DNA obtained in the above B. to a particulate carrier obtained in the above A. is conducted by a method comprising the following steps:
1) dissolve DNA fragments (a) and (b) (400 µg, each) into 1 M NaCl (250 µl);
2) heat the solution of the above 1) at 90°C on a water bath and allow to stand for about 5 minutes at the same temperature;
3) cool the solution of the above 2) to 35°C over one hour;
4) add 0.4 M NaHCO₃ (pH 7.5) (250 µl) to the solution of the above 3) and mix thoroughly;
5) add tresyl-activated silica gel (250 mg) to the mixture of the above 4) and mix thoroughly;
6) stir the mixture of the above 5) at room temperature for 18 hours; and
7) prepare two types of DNA-immobilized particulate carriers in the following manner.

The mixture of the above 6) is divided into two parts and one of them is washed 3 times with a mixture of 0.5 M NaCl and 0.2 M NaHCO₃ (pH 7.5) to yield a carrier to which a double-stranded DNA consisting (a) and (b) is immobilized.

The other half is used to obtain carriers to which single-stranded DNA (a) and (b) is attached separately. Thus, the mixture is placed at 95°C for 5 minutes and rapidly centrifuged (12,000 rpm x 1 min) to separate the supernatant. To the solid phase are added 300 µl of 0.5 M NaCl and 0.2 M NaHCO₃ (pH 7.5), and the mixture is treated twice by the process consisting of 5 minutes-heating at 95°C and separation of supernatant by centrifugation, to give particulate carrier to which single-stranded DNA is attached. This type of carrier is mostly a DNA (a)-immobilized carrier.

The same procedure as the above is repeated using DNA fragments (c) and (d), and (e) and (f), respectively, and obtained two types of carriers for each pair of DNAs. Accordingly, there obtained six types of DNA-immobilized carriers including three double-stranded DNA-immobilized carriers and three single-stranded DNA-immobilized carriers for three pairs of DNAs, that is (a) and (b), (c) and (d), and (e) and (f).

### 2. Detection of Anti-DNA Antibody

Effectiveness of the DNA-immobilized particles obtained above in the detection of anti-DNA antibody was evaluated as follows.

As the specific anti-DNA antibody, anti-DNA monoclonal antibody was used. For comparison, non-specific antibodies, i.e., immunoglobulin G (IgG) and Immunoglobulin M (IgM) were used. The control trial was conducted using a sample without any antibodies.

The anti-DNA monoclonal antibody used in the present experiment was obtained by the process disclosed in the literature (for example, T. Koike et al; Immunology Letter 4 93 (1982)) and the details of the process are well known to one of skill. An auto-immune mouse having anti-DNA antibody producing cells is then sacrificed and spleen cells obtained are fused with myeloma cells, which result in hybrid cells referred to as hybridomas. From the hybridomas, a desired clone which secretes a single antibody species to the DNA is isolated so as to obtain the specific monoclonal antibody.

The assay, which uses the above DNA-immobilized carrier (prepared in the above 1., C.), is carried out as follows:
a) add DNA-immobilized silica gel to a 96-well microtiter plate at a concentration of about 5 x 10⁵/well;
b) wash the gel with 200 µl of buffer A [1% BSA (bovine serum albumin), 0.05 % Tween 20, 0.1 % NaN₃ and 100 mM Tris-HCl (pH 7.4)];
c) add 30 µl of a dilution (1 µg/ml) of anti-DNA monoclonal antibody to each well and allow the plate to stand for 60 minutes at room temperature;
d) wash the plate three times with buffer A;
e) add 20 µl of a labeled secondary antibody, a 1/150 dilution of goat anti-mouse IgG + IgM + FITC (TAGO, Co., Ltd.), to each well and allow the plate to stand for 30 minutes at room temperature;
f) wash the plate twice with buffer A;
g) add 400 µl of 1 % trifluoroacetic acid and 10 mM phosphate buffer to each well and mix thoroughly and transfer the content of the well to a test tube separately to obtain samples; and
h) detect the signal present on particles in each sample by flow cytometry using FACS tar (one of FACS series).

The above procedures were repeated using IgG or IgM instead of anti-DNA monoclonal antibody in the step c), and in the absence of antibody. Results are shown in the accompanying drawings, Figures 1 to 6.

In the figures, the number of particles are plotted on the ordinate and the level of fluorescence on the axis. The solid lines represent the detection pattern obtained in the experiments conducted using antibody-containing samples and the dotted lines represent those obtained in the control experiments conducted using antibody-free samples.

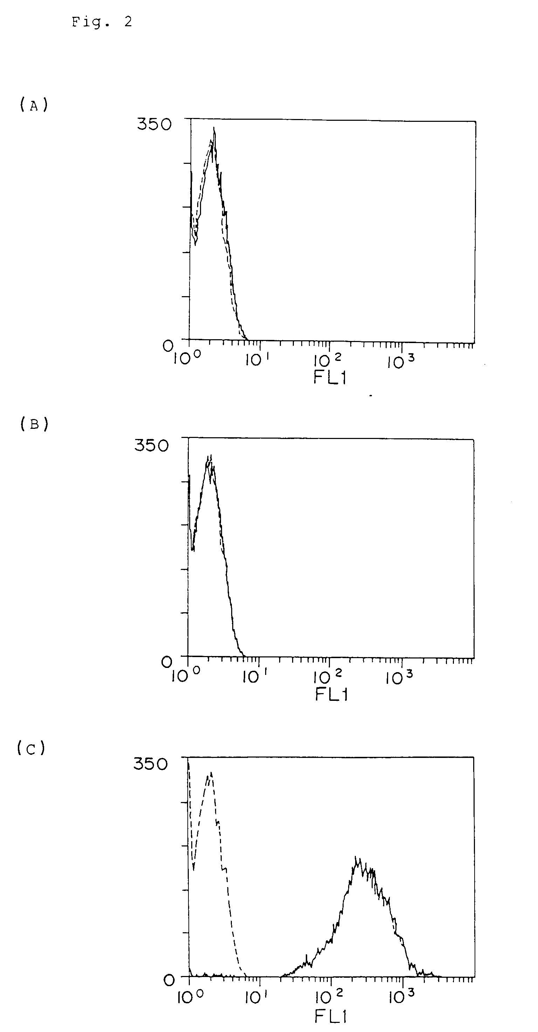

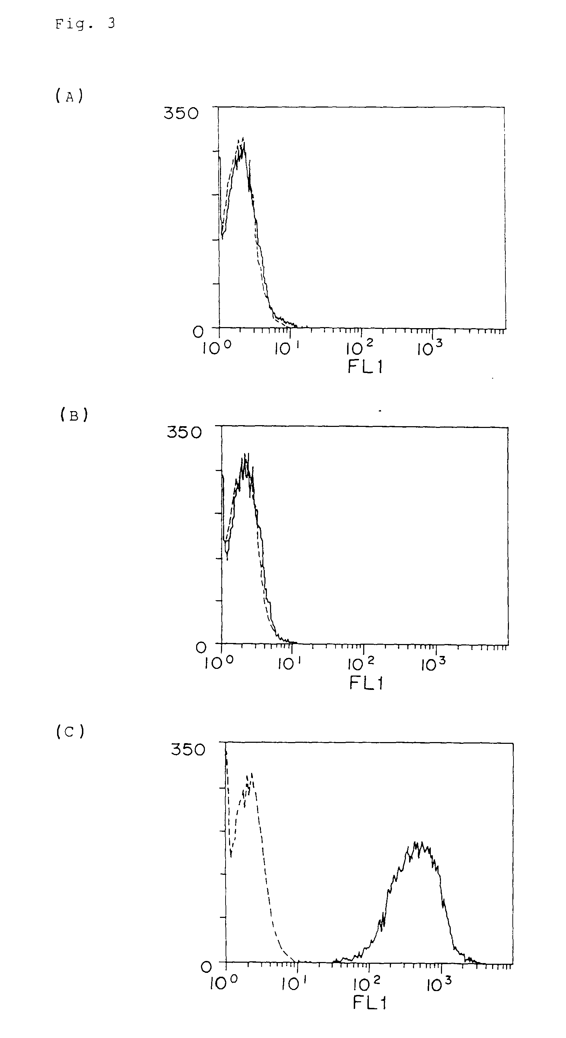

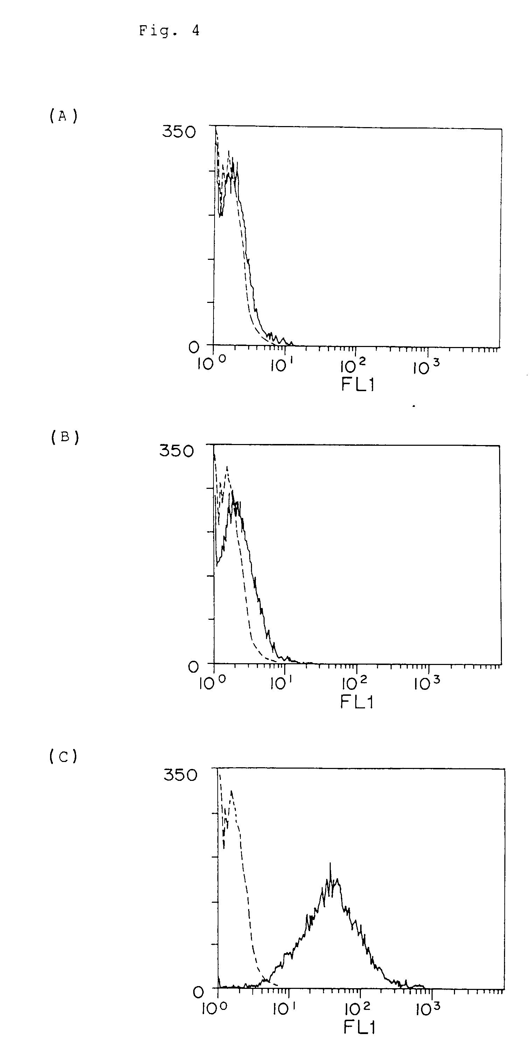

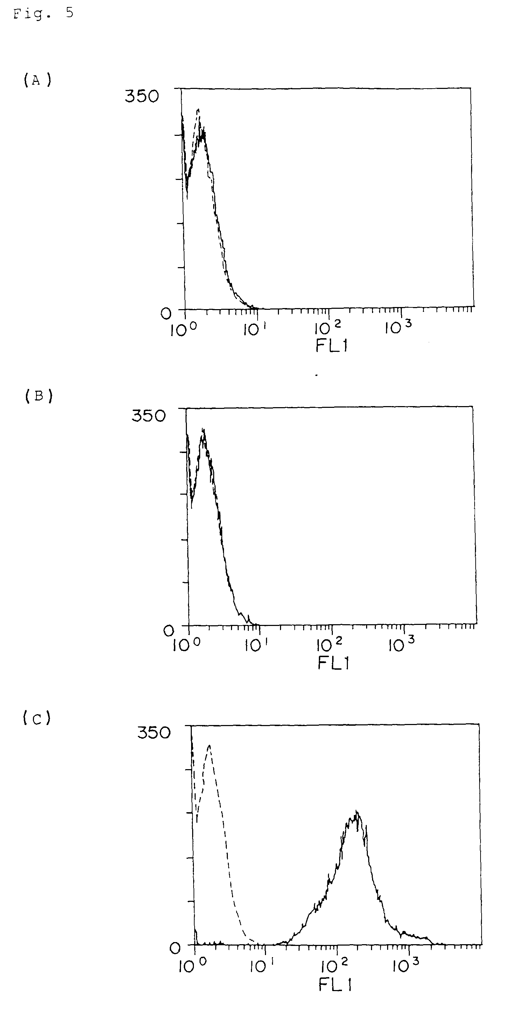

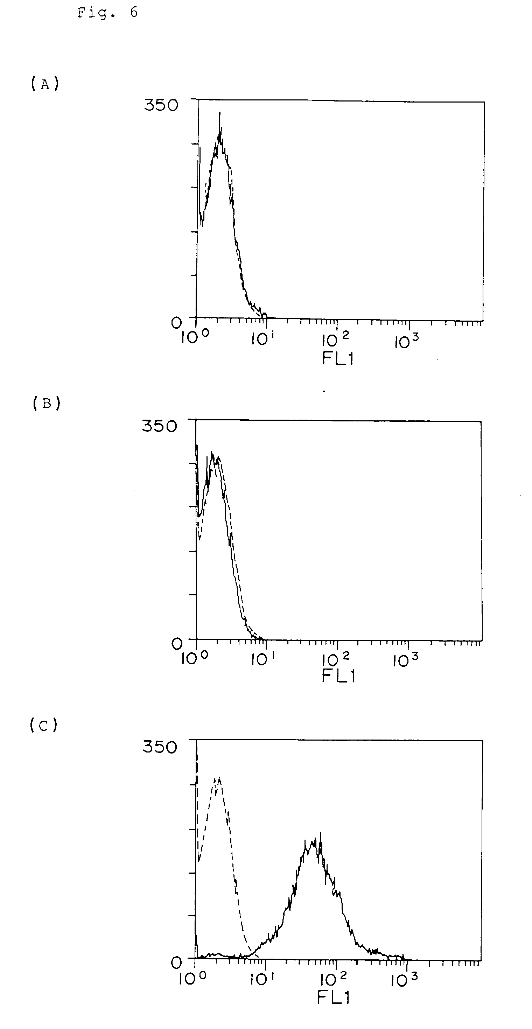

Figures 1 and 2 show the results obtained using carriers to which DNA (a) and (b) are immobilized as single- or double-stranded DNA, respectively. Figures 3 and 4 show the results obtained using carriers to which DNA (c) and (d) are immobilized as single- or double-stranded DNA, respectively. Figures 5 and 6 show the results obtained using carriers to which DNA (e) and (f) are immobilized as single- or double-stranded DNA, respectively. Throughout the Figures 1 to 6, the panel A depicts the binding effect of IgG, the panel B depicts that of IgM, and the panel C depicts that of anti-DNA monoclonal antibody. The order of the number of particles shown on the ordinate is 10,000.

When the DNA immobilized on a particle is effectively bound to antibody to be detected, the level of fluorescence of said particle increases, and the peak, as the total fluorescence of all the particles detected, should shift to the right. The extent of the shift indicates the facility or strength of binding between the immobilized substance (antigen) and other substance to be detected (antibody), in other words, it indicates the affinity of these substances.

From Figures 1 to 6, it can be easily seen that neither of the non-specific antibodies IgG and IgM, which lack the affinity to DNA, causes the shift of peak (panels A and B, respectively), whereas the specific monoclonal antibody causes a significant shift of peak (panel C). This demonstrates that the present method is effective for the detection of substance A which is specific to an immobilized substance B on microparticles.

In the present assay, the variation of data, that is, the variation of level of fluorescence from particle to particle, is reflected as the breadth of peak. Further, each particle can be regarded to be equivalent to one well of the currently used ELISA. Therefore, according to the present method, about 10⁴ to 10⁵ times of data can be obtained by only one experiment in comparison with ELISA. The data expressed as a peak are highly reliable and allow one-to evaluate the affinity between two substances, for example, an antigen and antibody, correctly and efficiently.

### Example 2

### LYSOZYME-IMMOBILIZED CARRIER

### 1. Preparation of Lysozyme-Immobilized Particulate Carriers

A. As a solid carrier for the immobilization of lysozyme, SIMPACK diol 300 was activated with tresyl chloride in the same manner as described in Example 1, 1. A. The tresyl-activated silica gel was stored in a Kjeldahl flask until use.

Lysozyme (Funakoshi & Co. Ltd.) was immobilized as follows:
1) dissolve lysozyme (1 mg) into 500 µl of 0.5 M NaCl and 0.2 M NaHCO₃ (pH 7.5);
2) add 250 mg of tresyl-activated silica gel to the solution of the above 1) and mix thoroughly;
3) stir the mixture. of the above 3) at room temperature for 18 hours;
4) wash the resultant silica gel three times with 0.5 M NaCl and 0.2 M NaHCO₃ (pH 7.5);

Thus obtained lysozyme-immobilized microparticles is then used for detecting the anti-lysozyme antibody as follows.

### 2. Detection of Anti-lysozyme Antibody

The evaluation of the effectiveness of the lysozyme-immobilized particulate carriers in the assay of anti-lysozyme antibody was conducted in the similar manner as described in the above Example 1.

The lysozeme-specific monoclonal antibody was prepared from hybridoma obtained by fusion of spleen cells of lysozeme-immunized mouse and mouse myeloma cells. Immunoglobulins G (IgG) and M (IgM) were also used and the control trial carried out using a sample without any antibodies.

The assay was carried out in accordance with the procedures described in Example 1. Thus, to a 96-well microtiter plate was added lysozyme-immobilized silica gel at a concentration of about 5 x 10⁵/well and washed with 200 µl of buffer A. After addition of 30 µl of a solution (1 µg/ml) of anti-lysozyme monoclonal antibody into each well, the plate was allowed to stand for 60 minutes at room temperature. After washing the plate with buffer A (x3), 20 µl of a secondary antibody solution, 1/150 dilution of a goat anti-mouse IgG + IgM + FIFTC (TAGO, Co., Ltd.) was added to each well and the plate was allowed to stand for 30 minutes at room temperature. The plate was then washed twice with buffer A. To each well were added 400 µl of 1 % trifluoroacetic acid and 10 mM phosphate buffer and mixed thoroughly. The content of each well was transferred into a test tube to give samples. Each sample was subjected to flow cytometry for the detection of signals present on the micropraticles using FACS tar.

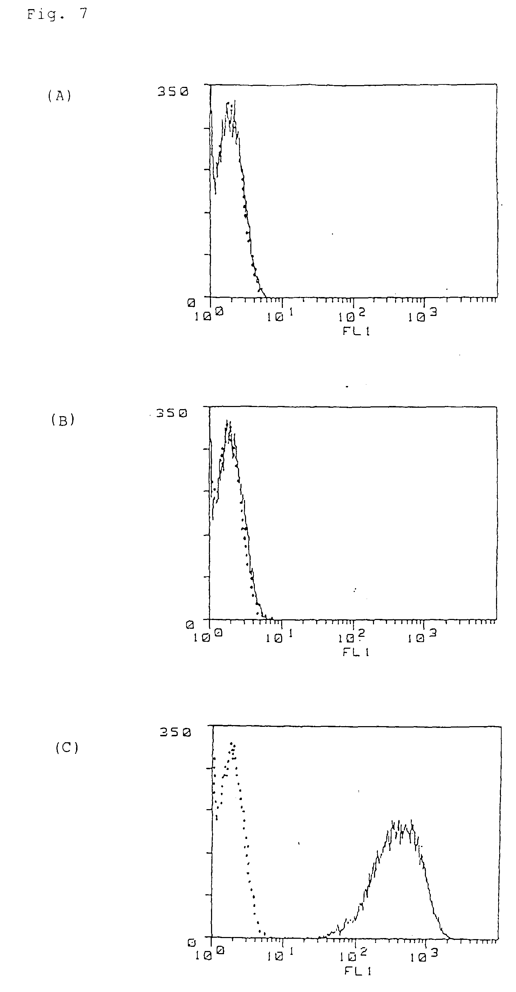

The above procedure was repeated using IgG or IgM instead of anti-lysozyme monoclonal antibody, and in the absence of antibody. Results are shown in the accompanying drawing, Figure 7.

In Figure 7, the number of particles are plotted on the ordinate and the level of fluorescence on the abscissa. The solid lines represent the detection pattern obtained in the experiments using antibody-containing samples and the dotted lines represent those obtained in control experiments using an antibody-free sample. The panel A depicts the binding effect of IgG, the panel B depicts the binding effect of IgM and the panel C depicts the binding effect of anti-lysozyme monoclonal antibody. The order of number of particles shown on the ordinate is 10,000.

When the lysozyme immobilized on a particle is effectively bound to antibody to be detected, the level of fluorescence should increases, and-the peak, as the total fluorescence of all the particles detected, should shifts to the right. The extent of the shift of the peak indicates the facility or strength of binding between immobilized lysozyme (antigen) and anti-lysozyme antibody, in other words, it indicates the affinity of these substances.

From Figure 7, it can be easily seen that neither of non-specific antibodies IgG and IgM causes the shift of peak (panels A and B), whereas the specific anti-lysozyme monoclonal antibody causes a significant shift of peak (panel C). The variation of data, that is, the variation of level of fluorescence from particle to particle, is reflected as the breadth of peak. Thus, the present assay, where each particle can be regarded as a well in ELISA, gives data as many as almost 10,000 times of those obtained by ELISA. The data obtained by the present assay are highly reliable, and it is possible to evaluate the affinity between an antigen and antibody more correctly.

The Example also demonstrates that the present method gives data with high reliability and is useful for the detection of a substance in a sample and evaluation of the affinity between an antigen and antibody correctly and efficiently.

### Example 3

### DNA-IMMOBILIZED CARRIER

### 1. Preparation of DNA-Immobilized Particulate Carriers

As a solid carrier for the immobilization of DNA, a commercially available particulate organic polymer, Tresyl 5PW (TOSO, & Co. Ltd.), was used.

The following 27-mer DNA-fragment (a) to be immobilized and its complementary sequence (b) were synthesized using DNA Synthesizer A-391EP PCR MATE (ABI, Inc.).

5′ X ACT TTC TGT TGC TTC ATC TTT GCA GAG 3′ (a)

5′ CTC TGC AAA GAT GAA GCA ACA GAA AGT 3′ (b)

Immobilization of the above DNA fragments were conducted in the same manner as described in Example 1, 1. C. except that Tresyl 5PW (250 mg) was used instead of SIMPACK diol and two types of DNA-immobilized carriers, a carrier to which double-stranded DNA consisting of (a) and (b) have been immobilized and a mixture of carriers to which the single-stranded DNA (a) and (b) are immobilized, separately.

### 2. Detection of Anti-DNA Antibody

The evaluation of the effectiveness of the DNA-immobilized particulate carriers in the assay of anti-DNA antibody was conducted in the same manner as described in the above Example 1, 2.

The DNA-specific monoclonal antibody was prepared as mentioned above. As non-specific antibody, IgG was used.

The assay was carried out in accordance with the procedures described in Example 1, 2. Thus, to a 96-well microtiter plate was added DNA-immobilized silica gel at a concentration of about 5 x 10⁵/well, and the plate was washed with 200 µl of buffer A. After addition of 30 µl of a solution (1 µg/ml) of anti-DNA monoclonal antibody into each well, the plate was allowed to stand for 60 minutes at room temperature. After washing the plate with buffer A (x3), 20 µl of a secondary antibody solution, 1/150 dilution of a goat anti-mouse IgG + IgM + FIFTC (TAGO, Co., Ltd.), was added to each well and the plate was allowed to stand for 30 minutes at room temperature. The plate was-then washed twice with buffer A. To each well were added 400 µl of 1 % trifluoroacetic acid and 10 mM phosphate buffer, and they were mixed thoroughly. The content of each well was transferred into a test tube to give samples. Each sample was subjected to flow cytometry for the detection of signals present on the micropraticles using FACS tar.

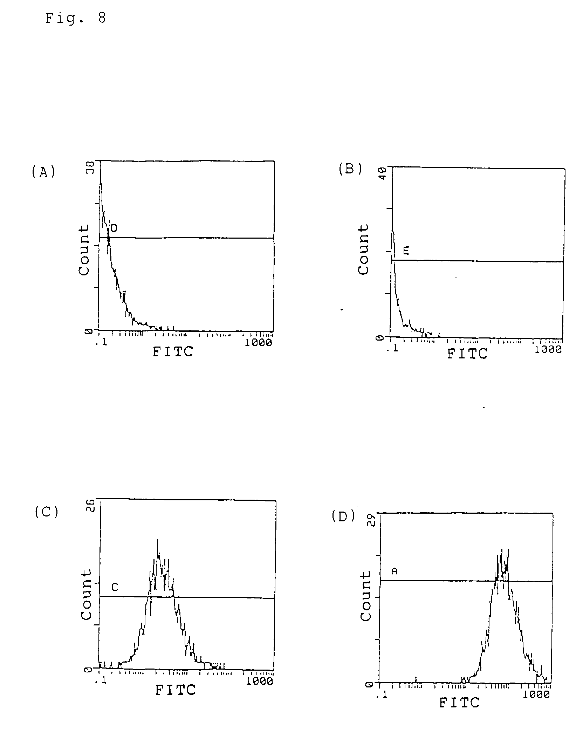

The above procedure was repeated using IgG instead of anti-DNA monoclonal antibody. Results are shown in the accompanying drawings, Figure 8.

In Figure 8, the number of particles are plotted on the ordinate and the level of fluorescence on the abscissa. The panels A and C show the results obtained using carriers to which DNA (a) and (b) are immobilized as single-stranded DNA, while the panels (B) and (D) show the results obtained using carriers to which double-stranded DNA are immobilized. The panels A and B depict the binding effect of IgG and the panels C and D depict the binding effect of anti-DNA monoclonal antibody. The order of number of particles shown on the ordinate is 10,000.

When the DNA immobilized on a particle is effectively bound to antibody to be detected, the level of fluorescence should increase, and the peak, as the total fluorescence of all the particles detected, should shift to the right. The extent of the shift of the peak indicates the facility or strength of binding between immobilized DNA (antigen) and anti-DNA antibody, in other words, it indicates the affinity of these substances.

From Figure 8, it can be easily seen that the non-specific antibody IgG does not cause the shift of peak (panels A and B), whereas the specific anti-DNA monoclonal antibody causes a significant shift of peak (panels C and D). The variation of data, that is, the variation of level of fluorescence from particle to particle, is reflected as the breadth of peak. Thus, the present assay, where each particle can be regarded as a well in ELISA, gives data as many as almost 10,000 times of those obtained by ELISA. The data obtained by the present assay are highly reliable, and it is possible to evaluate the affinity between an antigen and antibody more correctly.

The Example also demonstrates that the present method gives data with high reliability and is useful for the detection of a substance in a sample and evaluation of the affinity between an antigen and antibody correctly and efficiently.

## Claims

1. A method for qualitatively or quantitatively measuring an antibody A in a sample solution, which comprises the steps of:
immobilizing an antigen B capable of specifically binding with antibody A to a particulate carrier comprising silica gel;
contacting said particulate carrier to which the antigen B has been immobilized with a sample solution containing the antibody A, thereby forming a complex comprising antibody A, antigen B and said carrier; and
adding a labeled secondary antibody C capable of binding antibody A to the sample solution; and
detecting said antibody A bound to the particulate carrier through the antigen B by means of flow cytometry.

## Patentansprüche

1. Ein Verfahren für die qualitative und quantitative Messung eines Antikörpers A in einer Probenlösung, umfassend die Stufen:
Immobilisieren eines Antigens B, das zu einer spezifischen Bindung mit dem Antikörper A fähig ist, an einen aus Partikeln bestehenden Träger, der Silicagel umfaßt;
Zusammenbringen des genannten aus Partikeln bestehenden Trägers, auf welchem das Antigen B immobilisiert worden ist, mit einer den Antikörper A enthaltenden Probenlösung unter Bildung eines Komplexes, welcher aus dem Antikörper A, dem Antigen B und dem genannten Träger besteht; und
Zugabe eines markierten sekundären Antikörpers C, der fähig ist, den Antikörper A aus der Probenlösung zu binden; und
Nachweis des genannten Antikörpers A, der an den aus Partikeln bestehenden Träger über das Antigen B gebunden ist, mittels Durchflußzytometrie.

## Revendications

1. Procédé de dosage qualitatif ou quantitatif d'un anticorps A dans une solution d'échantillon, qui comprend les étapes selon lesquelles:
on immobilise sur un support particulaire comprenant du gel de silice un antigène B capable de se lier de façon spécifique à l'anticorps A;
on met en contact ledit support particulaire sur lequel a été immobilisé l'antigène B avec une solution d'échantillon contenant l'anticorps A, en formant ainsi un complexe comprenant l'anticorps A, l'antigène B et ledit support; et
on ajoute à la solution d'échantillon un second anticorps C marqué capable de se lier à l'anticorps A; et
on décèle au moyen d'une cytométrie en flux ledit anticorps A lié au support particulaire par l'intermédiaire de l'antigène B.
